Europäisches Patentamt

European Patent Office

Office européen des brevets

(11) Publication number: 0 498 999 A2

# EUROPEAN PATENT APPLICATION

(21) Application number: 91311861.8

(22) Date of filing: 20.12.91

(51) Int. Cl.5: G01N 33/49, G01N 33/497

(30) Priority: 11.01.91 GB 9100674

(43) Date of publication of application:
19.08.92 Bulletin 92/34

(84) Designated Contracting States:
CH DE ES FR IT LI NL

(71) Applicant: GEC-Marconi Limited
The Grove Warren Lane
Stanmore Middlesex HA7 4LY(GB)

(72) Inventor: Perera, Guruge Elmo Lakshman
37 Meadow Way
Wembley, Middlesex HA9 7LB(GB)

(74) Representative: George, Sidney Arthur
The General Electric Company p.l.c. GEC
Patent Department(Wembley Office) Hirst
Research Centre East Lane
Wembley Middlesex, HA9 7PP(GB)

(54) Monitoring apparatus.

(57) In gas monitoring apparatus for use, for example, in monitoring the gas content of blood, the pressure and the concentration of the gas sample are increased, and the pressurised and concentrated gas is fed to gas detectors. The effective volume of a micron-sized chamber (3,15,23) is varied by means of a piezoelectric actuator, or other actuator device, in order to draw the gas into the chamber and to pressurise the gas.

FIGURE 1

This invention relates to monitoring apparatus, and particularly to apparatus for sampling gas, for use, for example, in monitoring the gas content of blood, in mass spectrometry and in forensic gas detection.

Continuous and precise monitoring of arterial oxygenation is important in the management of critically-ill patients. Currently, the monitoring is effected by oxymetry using both invasive and non-invasive devices. Comprehensive blood gas monitoring ($O_2$,$CO_2$ and pH) and treatment are carried out by Eximer Membrane Oxygenerators (ECMO machines) during cardiac surgery. Such machines are not normally used as continuous blood gas monitoring systems.

The pulse oxymeter is a non-invasive device. In use of the device, the saturation and pulse rate are measured via a single sensor, which is normally placed on an extremity of the body. This provides real-time readings and is independent of peripheral perfusion except in extremely low output conditions. However, a malposition sensor attachment to the device has presented frequent problems, especially in young and restless patients. The pulse oxymeter is also known to present inaccurate data in low output conditions, and it is therefore sometimes necessary to correlate the output with pulse readings.

The other most frequently used non-invasive device for measuring arterial oxygenation is the transcutaneous monitor (tcpO$_2$ monitor). The combined tcpO$_2$/pCO$_2$ electrode combines a heating element, two temperature sensors, a Clark-type oxygen electrode and a Severinghaus-type carbon dioxide electrode in a single unit. The temperature of the electrode is sensed by a negative temperature coefficient (NTC) resistor incorporated in an Ag/AgCl reference electrode. Due to the high thermal conductivity of the silver body, the NTC resistor responds quickly to any changes in temperature. A thermostat maintains the electrode at the present temperature. When the electrode is attached to the skin, the generated heat is transferred from the heating element via the silver body to the skin surface. The heating produces local vasodilation and increases the permeability of the oxygen and carbon dioxide, rendering a surface measurement possible. This allows continuous measurements to be made, but response time varies, frequent calibration is necessary and change of site is essential to prevent skin damage. There could be poor correlation with partial pressure (or tension) of oxygen, depending on the age of the patient, the site of the electrode, oxygen levels, cardiac output peripheral perfusion and the condition of the skin. This can cause problems when tcpO$_2$ monitors are used in pediatric intensive care cases.

The present tcpO$_2$ monitors are arranged to heat the surface of the skin up to $44°C$. This temperature is found to be the optimum point at which enough $O_2$ and $CO_2$ molecules can be extracted from the skin without damaging the patient. Even at $44°C$ it is recommended that the siting of the electrodes be changed every four hours during continuous monitoring. In pediatric intensive care this is carried out at even more frequent time intervals. The present technique does not allow the skin temperature to be reduced down to the normal body temperature because the perfusion rate is low at normal temperatures.

It is an object of the present invention to provide an improved gas monitoring apparatus.

According to the invention there is provided gas monitoring apparatus comprising means to receive gas or gases to be monitored; and means for increasing the pressure and the concentration of the received gas or gases and for delivering the pressurised gas or gases to gas detection means.

Preferably the gas pressure increasing means comprises one or more micron-sized pump stages, the or each pump stage including, for example, a piezoelectric, electrostatic, electromagnetic or electrodynamic actuator.

Embodiments of the invention will now be described, by way of example, with reference to the accompanying drawings, in which

Figure 1 is a schematic diagram of a multistage gas sampler in accordance with the invention,

Figure 2 to 5 are schematic cross-sectional views of electrostatic, electromagnetic, electrodynamic and piezoelectric displacers, respectively, as examples of alternative pump devices for use in the gas sampler,

Figure 6 is a schematic pictorial view of a two-stage gas sampler in accordance with the invention, and

Figure 7(a),(b) and (c) are respective plan views of three wafers for forming a practical embodiment of the gas sampler of Figure 6.

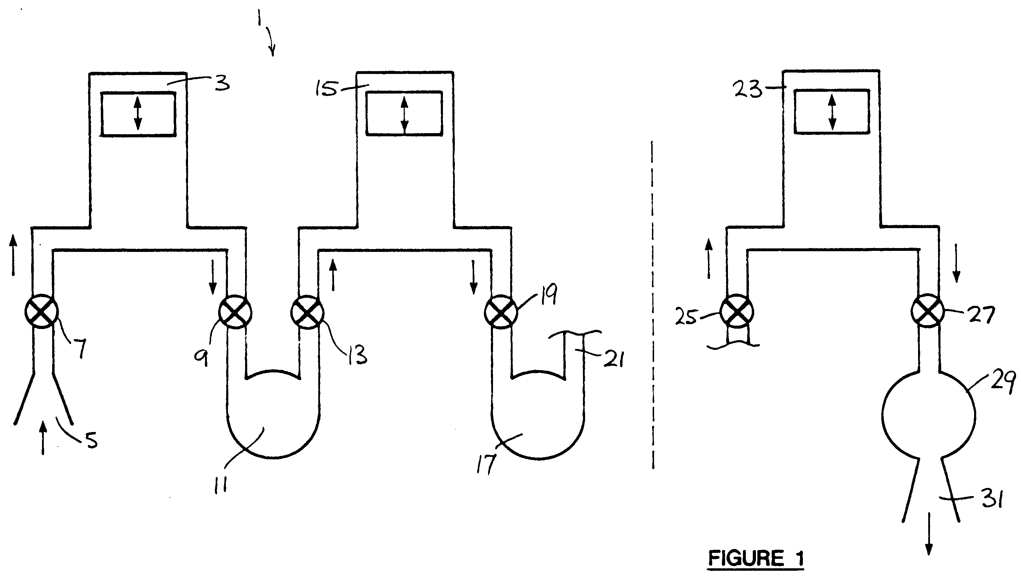

Referring to Figure 1, a gas sampler 1 comprises a positive displacement pump 3 which draws gas to be sampled into an inlet 5 and through a non-return valve 7. The gas is compressed in the pump and passes out, through a non-return valve 9, into a reservoir 11. From there, the gas passes through a non-return valve 13 and into a pump 15. The pump 15 further compresses the gas and feeds it into a reservoir 17 via a non-return valve 19. The gas leaves the reservoir 17 via an outlet 21 and passes through as many further compression stages as necessary, until it reaches an output stage pump 23 via a non-return valve 25. The gas is finally compressed by the pump 23 and passes through a non-return valve 27 to a reservoir 29 and thence out through an outlet 31 to an oxymeter or other gas-monitoring device (not shown).

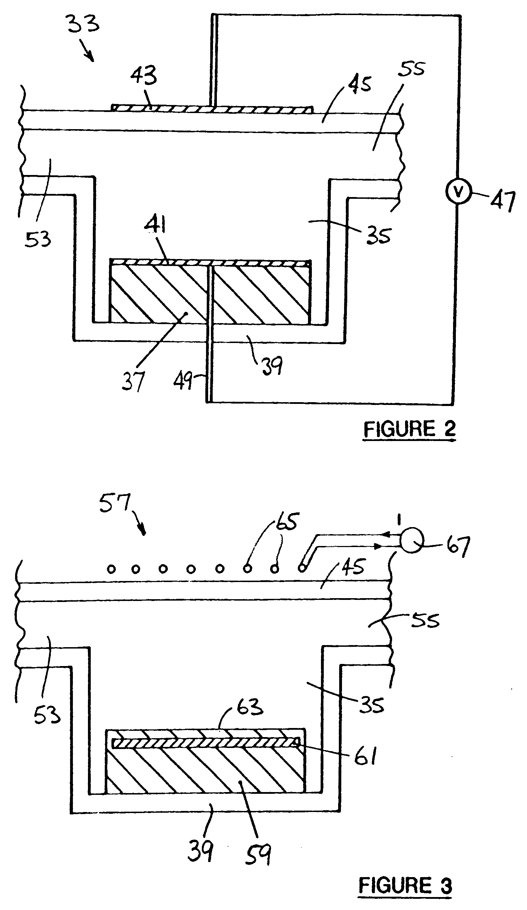

The pumps 3,15---23 may comprise any of a number of different types of positive displacement devices as shown, for example, schematically in Figures 2-5, in which corresponding components have the same reference numerals.

Referring to Figure 2, an electrostatic displacement device 33 comprises, within an electrically-insulating chamber 35, a body 37 of resilient material bonded by one major surface to the chamber wall 39. A metal film 41 is deposited on the opposite surface of the body 37. A second metal film 43 is deposited on the outside of the opposing wall 45 of the chamber. A voltage source 47 is connected, via conductors 49,51, to the films 41,43. When a potential difference is applied between the films, they will be mutually attracted, so that the film 41 will tend to move towards the film 43, stretching the body 37 and thereby reducing the effective volume of the chamber. When the potential difference is removed, the body will revert to its original shape. A pumping action is therefore produced, and this can be repeated as often as necessary by switching the supply on and off, or by using a pulse generator as the source 47. Gas will thereby be drawn into the chamber 35 via an inlet 53, compressed, and fed out of the chamber via an outlet 55.

Figure 3 shows an electromagnetic displacement device 57, in which a block 59 of resilient material is bonded by one major surface to the chamber wall 39. A layer 61 of magnetic or magnetisable material is embedded in, or attached to, the block 59 adjacent its opposite surface 63. A current loop arrangement 65 is located on or adjacent the wall 45, outside the chamber 35. The current loop arrangement is energisable from a source 67, so that the magnetic field produced thereby will cause displacement of the layer 61, thereby changing the effective volume of the chamber and causing pumping of the gas as the supply is switched.

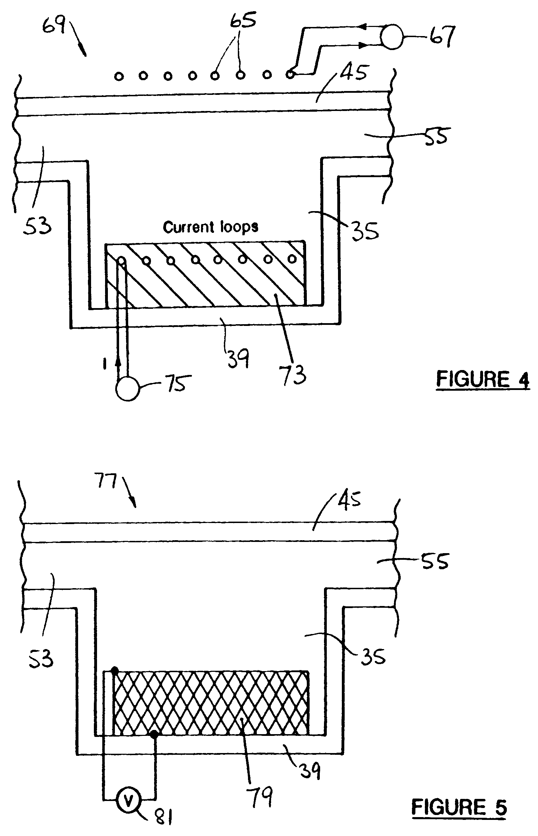

Figure 4 shows an electrodynamic displacement device 69, including a current loop arrangement 65, similar to that in Figure 3. In this case, a further current loop arrangement 71 is embedded in, or attached to, a block 73 of resilient material which is bonded to the wall 39. The current loop arrangements 65 and 71 are energised by respective current sources 67,75. Interaction of the magnetic fields produced by the loops causes displacement of the loop arrangement 71, and hence the resilient block, thereby changing the effective volume of the chamber 35 and causing pumping of the gas.

In a displacement device 77 as shown in Figure 5, the pumping is caused by deformation of a block 79 of piezoelectric material which is bonded to the wall 39. The deformation of the block is caused by the switching of a voltage applied thereto from a source 81.

The pressure increase obtained within a stage of the gas sampler depends upon the efficiency of the non-return valves, the frequency and the amplitude of displacement of the pump, the pressure at the inlet to the stage and the flow losses. For a given set of conditions, an electrodynamic displacer or an electro magnetic displacer is likely to displace more gas than an electrostatic displacer.

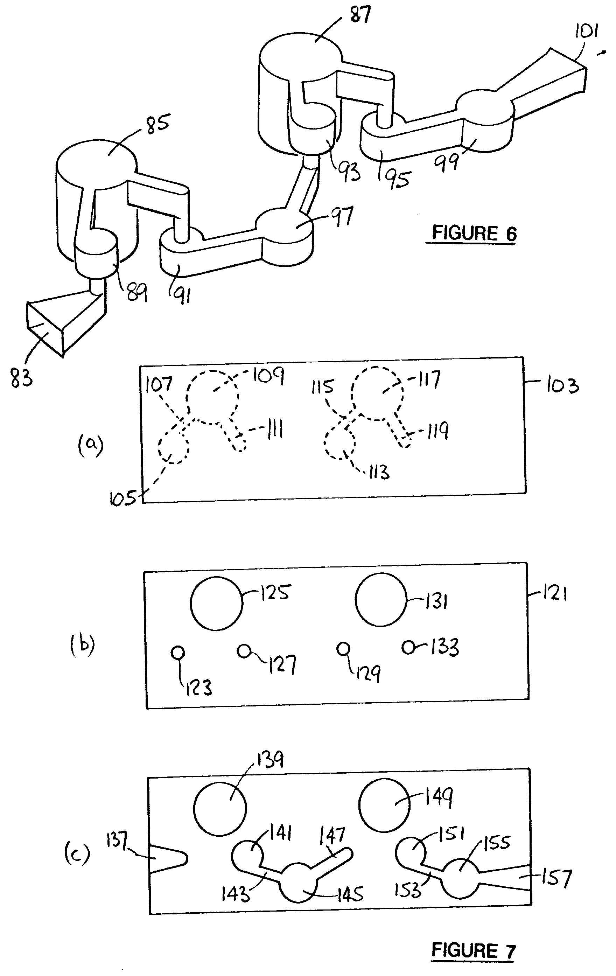

Figure 6 shows, schematically, a two-stage micro-sampler, and Figure 7 shows a method of constructing that micro-sampler. The device comprises an inlet 83, first and second stage pumps 85 and 87, respectively, non-return valves 89,91,93 and 95, reservoirs 97 and 99 and an outlet 101 which delivers the compressed gas to a monitoring device (not shown). The device is formed from three wafers of, for example, silicon. Figure 7(a) is a plan view of a top wafer 103 which has cavities 105 to 119 etched into its underside. A middle wafer 121 (Figure 7(b)) has apertures 123-133 etched therethrough. A bottom wafer 135 (Figure 7-(c)) has cavities 137-157 etched into its upper surface. The cavities 137 and 157 extend to the end edges of the wafer 135 to form the inlet 83 and the outlet 101, respectively.

When the wafers are assembled together, the cavity 105, the aperture 123 and the cavity 137 communicate with each other. The cavity 105 is provided with a non-return valve (not shown) such as a fluidic valve or preferably a vortex diode valve, to form the valve 89 of Figure 6. The cavity 109, the aperture 125 and the cavity 139 provide a housing for a displacer (not shown), which may be of any of the types described above, to form the pump 85. The cavity 111, the aperture 127 and the cavity 141 are interconnected, the cavity 141 housing the non-return valve 91. The cavity 145 forms the reservoir 97. The cavity 147, the aperture 129 and the cavity 113 are interconnnected, and the cavity 113 contains the valve 93. The cavity 117, the aperture 131 and the cavity 145 house a displacer (not shown), as above, to form the pump 87. The cavity 119, the aperture 133 and the cavity 151 are interconnected, and the cavity 151 houses the valve 95. The cavity 155 forms the reservoir 99.

The cavities and the apertures are preferably all of micron dimensions, produced by micro-machining techniques. For example, the non-return valve cavities 105,113,141 an 151 may be of the order of 1000um diameter.

Gas monitoring apparatus according to the invention is particularly applicable to the feeding of gas or gases to a detector where the concentration of the available gas is low.

The pressure P, the number of molecules per unit volume n/V (i.e. the concentration of the gas), the universal gas constant R and the temperature T are related by the equation $P = \frac{n}{V}.RT$.

It will be apparent, therefore, that for a constant temperature the concentration will increase with the pressure.

Where there is a mixture of gases, the individual gases may be separated, and each gas may be pressurised and concentrated by a respective apparatus in accordance with the invention.

The provision of the pump stages of the present invention to increase the gas pressure allows the transport of saturated $O_2$ and $CO_2$ molecules at normal temperature to the gas detector (e.g. oxymeter) membranes, so that the probe formed by the multistage gas sampler can operate at normal body temperature. The fear of damage to the skin which arises from increasing the temperature to 44°C is therefore avoided. By increasing the flow of $O_2$ and $CO_2$ through the detector membranes, the detector can be calibrated to allow better control over its response time.

Although pumps comprising deformable resilient blocks have been proposed in the above embodiments, other types of pump stages might alternatively be used to provide the gas pressure and flow rate required by a transcutaneous oxymeter.

The gas detector might alternatively be, for example, a mass spectrometer or a forensic gas detector.

It is considered that the addition of the pump stage or stages will add only a relatively small amount to the total cost of a conventional detector.

## Claims

1. Gas monitoring apparatus characterised by means (5) to receive gas or gases to be monitored; and means (3,15,23) for increasing the pressure and the concentration of the received gas or gases and for delivering the pressurised and concentrated gas or gases to gas detection means.

2. Apparatus as claimed in Claim 1, characterised in that the means (3,15,23) for increasing the pressure comprises at least one chamber into which the gas is drawn; and means for changing the effective volume of the chamber.

3. Apparatus as claimed in Claim 2, characterised in that the volume changing means (3,15,23) comprises a piezoelectric actuator.

4. Apparatus as claimed in Claim 2, characterised in that the volume changing means (3,15,23) comprises an electrostatic actuator.

5. Apparatus as claimed in Claim 2, characterised in that the volume changing means (3,15,23) comprises an electromagnetic actuator.

6. Apparatus as claimed in Claim 2, characterised in that the volume changing means (3,15,23) comprises an electrodynamic actuator.

7. Apparatus as claimed in any preceding claim, characterised in that the means for increasing the pressure comprises a plurality of gas compression stages (3,15,23) connnected in series.

8. Apparatus as claimed in Claim 7, characterised in that each gas compression stage (3,15,23) includes an inlet path, an outlet path, non-return valve means (7,13,25) in the inlet path and non-return valve means (9,19,27) in the outlet path.

9. Apparatus as claimed in Claim 8, characterised in that the gas compression stages (3,15,23) and the valve means (7,9,13,19,25,27) are formed in a common unit.

10. Apparatus as claimed in Claim 9, characterised by reservoir means (11,17) between the compression stages (3,15,23), the reservoir means also being formed in said common unit.

11. Apparatus as claimed in Claim 10, characterised in that the unit is formed of at least one substrate in which cavities and/or apertures are provided for forming the compression stages (3,15,23), the reservoir means and the valve means (7,9,13,19,25,27).

12. Apparatus as claimed in Claim 10, characterised in that the cavities and/or apertures are of micron dimensions.

13. An oxymeter including apparatus as claimed in any preceding claim.

FIGURE 1

FIGURE 2

FIGURE 3

Current loops

**FIGURE 4**

69, 65, 67, 45, 55, 53, 35, 39, 73, 75, I

**FIGURE 5**

77, 45, 55, 53, 35, 39, 79, 81, V

FIGURE 6

(a)

(b)

(c)

FIGURE 7